(19) **Europäisches Patentamt**
**European Patent Office**
**Office européen des brevets**

(11) **EP 3 960 055 A1**

(12) **EUROPEAN PATENT APPLICATION**
published in accordance with Art. 153(4) EPC

(43) Date of publication:
**02.03.2022 Bulletin 2022/09**

(21) Application number: **21786077.4**

(22) Date of filing: **04.03.2021**

(51) Int Cl.:
*A47K 17/00* (2006.01)    *A61L 2/10* (2006.01)
*E03D 9/00* (2006.01)

(86) International application number:
**PCT/JP2021/008462**

(87) International publication number:
**WO 2022/014087 (20.01.2022 Gazette 2022/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.07.2020 JP 2020119894**

(71) Applicant: **Ushio Denki Kabushiki Kaisha**
**Tokyo 100-8150 (JP)**

(72) Inventors:
• **IGARASHI Tatsushi**
  **Tokyo**
  **1008150 (JP)**

• **OHASHI Hiroyuki**
  **Tokyo**
  **1008150 (JP)**
• **OKUMURA Yoshihiko**
  **Tokyo**
  **1008150 (JP)**
• **IMAMURA Atsushi**
  **Tokyo**
  **1008150 (JP)**

(74) Representative: **Tomerius, Isabel**
**Lang & Tomerius**
**Patentanwaltspartnerschaft mbB**
**Rosa-Bavarese-Strasse 5**
**80639 München (DE)**

(54) **INACTIVATION DEVICE AND INACTIVATION METHOD**

(57)    An inactivation device for inactivating harmful microorganisms and/or viruses is provided. The inactivation device includes: an ultraviolet light irradiation unit that irradiates an enclosed space where a human can enter and leave with light containing ultraviolet light having a wavelength of inactivating the microorganisms and/or viruses that are harmful to the human body; a sensor that detects presence of the human in the enclosed space; and a control unit that controls irradiation and non-irradiation with the light from the ultraviolet light irradiation unit based on a detection signal from the sensor. The control unit controls the ultraviolet light irradiation unit to irradiate a space including the human with the light for a predetermined time in accordance with the wavelength of the ultraviolet light contained in the light radiated from the ultraviolet light irradiation unit, during a period in which the human is determined to be present in the enclosed space based on the detection signal from the sensor.

FIG. 1

## Description

TECHNICAL FIELD

The present invention relates to an inactivation device and an inactivation method to inactivate harmful microorganisms and viruses.

BACKGROUND ART

Facilities where people frequently gather, such as medical facilities, schools, and government offices, are places where harmful microorganisms (bacteria, molds, etc.) and viruses can easily proliferate. These harmful microorganisms and viruses are especially prone to proliferate in confined spaces (enclosed spaces such as hospital rooms, restrooms, and elevators) in the above-mentioned facilities.

The above-mentioned harmful microorganisms proliferate on the floors, walls, and other surfaces in the above-mentioned confined spaces, or inside humans (or animals in some cases) entering or leaving the above-mentioned confined spaces, or the harmful microorganisms float in the above-mentioned confined spaces. This tendency is particularly pronounced in medical facilities. Specifically, infectious microorganisms derived from patients spread in confined spaces such as hospital rooms, restrooms in hospital rooms, and restrooms near outpatient reception areas. The spread infectious microorganisms adhere to the surfaces (floors, walls, etc.) constituting the confined spaces, or float in the spaces. As a result, the next person (such as another patient or visitor) entering the spaces (such as a restroom) can be infected, and in some cases, the infectious disease may spread in the medical facility.

In order to improve the situation described above, facilities where humans (and animals in some cases) gather (especially medical facilities) are required to take measures to decontaminate (sterilize) the above-mentioned harmful microorganisms (e.g., infectious microorganisms).

Patent Document 1 discloses a decontamination device that irradiates a space targeted for decontamination with ultraviolet light (UVC light) and decontaminates the space. This decontamination device radiates ultraviolet light into the space targeted for decontamination when it detects the absence of a person in the space targeted for decontamination.

Patent Document 2 discloses a system in which a motion sensor and a door sensor are mounted in an elevator, and the system radiates ultraviolet light for sterilization to the interior of the elevator when the above-mentioned sensors detect that no human is present in the elevator and the door is closed. Here, the radiated ultraviolet light has a wavelength of between approximately 240 nm and 280 nm.

CITATION LIST

PATENT DOCUMENTS

Patent Document 1: JP-A-2017-528258
Patent Document 2: US 2010/0032859A

SUMMARY OF INVENTION

Technical Problem

The proliferation or floating of harmful microorganisms in the confined spaces of the facilities is often caused by humans (patients) or animals with harmful microorganisms entering or leaving the above-mentioned spaces. Thus, for the efficient decontamination of such facilities, it is essential to decontaminate not only the surfaces and spaces inside the facilities, but also the surfaces of humans (patients) and animals present in the areas.

However, ultraviolet light having a wavelength suitable for decontamination has an adverse effect on humans or animals when directed thereto. Hence, Patent Document 1 and Patent Document 2 disclose decontamination systems using the irradiation with ultraviolet light, in which emission of ultraviolet light stops during the presence of humans in the irradiation area to ensure the safety of humans or animals.

Thus, the above-mentioned conventional decontamination systems cannot decontaminate the facilities efficiently. Also, the conventional decontamination systems cannot decontaminate the surfaces of humans (patients) and animals, thereby the area to be decontaminated needs to be broadened in consideration of the activity range of humans (patients) and animals.

It is an object of the present invention to provide an inactivation device and an inactivation method that can efficiently inactivate harmful microorganisms and/or viruses.

Solution to the problem

To solve the above-described problems, an inactivation device according to one aspect of the present invention for inactivating microorganisms and/or viruses that are harmful to a human body includes: an ultraviolet light irradiation unit that irradiates an enclosed space where a human can enter and leave with light containing ultraviolet light having a wavelength of inactivating the microorganisms and/or viruses that are harmful to the human body; a sensor that detects presence of the human in the enclosed space; and a control unit that controls irradiation and non-irradiation with the light from the ultraviolet light irradiation unit based on a detection signal from the sensor. The control unit controls the ultraviolet light irradiation unit to irradiate a space including the human with the light for a predetermined time in accordance with the wavelength of the ultraviolet light contained in the light radiated from the ultraviolet light irradiation unit, during a period in which the human is determined to be present in the enclosed space based on the detection signal from the sensor.

In this way, intentionally irradiating a human with light containing ultraviolet light is capable of inactivating at least either harmful microorganisms or viruses present on the surface of the human body (skin or surface of clothes). Hence, the irradiation prevents the harmful microorganisms or viruses attached to humans from spreading into an enclosed space. In addition, the irradiation prevents a human leaving the enclosed space from spreading the harmful microorganisms or viruses outside the enclosed space. Therefore, the irradiation avoids the expansion of the area to be decontaminated in the facility, resulting in efficient decontamination of the facility.

It is noted that the time of irradiating a human with ultraviolet light is determined in accordance with the wavelength of the ultraviolet light. The extent to which ultraviolet irradiation adversely affects to a human body depends on the wavelength of the ultraviolet light. Therefore, irradiating the human with ultraviolet light for the predetermined time in accordance with the wavelength of the ultraviolet light enables the efficient decontamination without adversely affecting to the human body.

In the above-described inactivation device, when the control unit determines that no human is present in the enclosed space based on the detection signal from the sensor, the control unit may control the ultraviolet irradiation unit to irradiate the enclosed space where no human is present with the light.

In this way, emission of light containing ultraviolet light to the enclosed space where no human is present is capable of efficiently inactivating at least part of the harmful microorganisms or viruses already present in the enclosed space, the harmful microorganisms or viruses spreading inside the enclosed space upon entering of the human into the enclosed space, the harmful microorganisms or viruses attached to a surface of the enclosed space upon contacting of the human with a part of the surface inside the enclosed space, or the harmful microorganisms or viruses floating in the flow of air inside the enclosed space upon entering of the human into the enclosed space.

In the above-described inactivation device, the control unit may control the ultraviolet irradiation unit to irradiate the enclosed space where no human is present with the light for a predetermined time, and to stop radiation of the light after the predetermined time.

This control enables a light source mounted in the ultraviolet irradiation unit to have a pause time, extending a service life of the light source.

In the above-described inactivation device, the sensor may include a first sensor that detects the human entering or leaving the enclosed space, and the control unit may control the ultraviolet light irradiation unit to irradiate the space including the human with the light for the predetermined time after the first sensor detects that the human has entered the enclosed space.

This control enables the human to be irradiated with light containing ultraviolet light immediately after the human enters the enclosed space, thereby efficiently suppressing the spread of harmful microorganisms or viruses from the human to the enclosed space.

In the above-described inactivation device, the first sensor may be at least one of a motion sensor that detects the presence or absence of a human in the enclosed space and a door sensor that detects the opening or closing of the door of the enclosed space.

This configuration enables easy and appropriate detection of the entering and exiting of a human into and out of the enclosed space.

In the above-described inactivation device, the sensor may include a second sensor that detects the presence of the human at a predetermined position in the enclosed space, and the control unit may control the ultraviolet light irradiation unit to irradiate the space including the human with the light for the predetermined time after the second sensor detects the presence of the human at the predetermined position in the enclosed space.

Thus, irradiating the human present in the predetermined position with light containing ultraviolet light enables ultraviolet light to be effectively radiated to the intended location on the surface of the human body.

In the above-described inactivation device, the sensor may include a first sensor that detects the human entering or leaving the enclosed space and a second sensor that detects the presence of the human present at a predetermined position in the enclosed space. The control unit may control the ultraviolet light irradiation unit to stop radiation of the light when the first sensor detects that the human has entered the enclosed space and start radiating the light to the space including the human for the predetermined time after the second sensor detects the presence of the human at the predetermined position in the enclosed space.

If the light containing the ultraviolet light is radiated to the enclosed space before a human enters, the control unit may control the ultraviolet light irradiation unit to temporarily stop irradiation with the ultraviolet light when a human enters the enclosed space. Once the human is in the predetermined position, the control unit may start irradiating the human with the light containing ultraviolet light for a predetermined time.

In the above-described inactivation device, the first sensor may be a door sensor that detects the opening or closing of a door of the enclosed space. If the second sensor does not detect the presence of the human at the predetermined position in the enclosed space, the control unit may control the ultraviolet light irradiation unit to radiate the light to the enclosed space where no human is present after a predetermined time has elapsed after the first sensor detects the opening or closing of the door.

This control enables an appropriate judgement that no human is present in the enclosed space, using both of the detection signal from the first sensor and the detection signal from the second sensor. For example, in case that the door unintentionally opens or closes due to forgotten locking or door defect, this configuration can prevent the inactivation device from mistakenly recognizing that the human has left the enclosed space and starting the irradiation with the ultraviolet light. Moreover, keeping ultraviolet irradiation from starting for a predetermined time after the opening or closing of the door can prevent the inactivation device from starting the irradiation with the ultraviolet light in the case that the human, after entering the enclosed space, is present at a position other than a predetermined position.

In the above-described inactivation device, the enclosed space may be a restroom stall and the second sensor may be a pressure sensor provided in a toilet seat.

In this case, the state that a person sits on the toilet seat of a toilet bowl in a restroom stall can be detected as a state that a person is present in a predetermined position. Since a movement of the human sitting on a toilet seat is relatively small, it is possible to effectively inactivate harmful microorganisms and viruses on the surface of the human body (skin and clothing).

In the above-described inactivation device, the ultraviolet irradiation unit may be mounted at a position such that the light is radiated to the human present at the predetermined position from a back of a head of the human.

This configuration prevents human's eyes from directly being exposed to the light containing ultraviolet light radiated from the ultraviolet light irradiation unit, thereby suppressing the occurrence of eye damage including eye pain, hyperemia, and corneal inflammation.

In the above-described inactivation device, the ultraviolet irradiation unit may be mounted at a position at which the light is radiated downward from an upper area of the enclosed space.

This configuration enables the irradiation with the light containing ultraviolet light over the entire enclosed space. Hence, it is possible to effectively inactivate harmful microorganisms and viruses attached to the walls or floors of the enclosed space.

In the above-described inactivation device, the predetermined time T1 (seconds) may be set to satisfy a formula as follows:

$$T1 \leq D_{max} / (W \times N)$$

where $D_{max}$ (mJ/cm$^2$) is an amount of maximum allowable ultraviolet light exposure to the human body in one day, W (mW/cm$^2$) is the irradiance of the ultraviolet light radiated on the surface of the human body, and N is the number of times the same person enters the enclosed space in one day.

Satisfying this formula enables the ultraviolet light having a wavelength suitable for inactivation of harmful microorganisms or viruses to be radiated to the human within the range of the amount of light that does not adversely affect a human body.

In the above-described inactivation device, an ultraviolet light irradiation operation of the ultraviolet light irradiation unit may include repetition of the ultraviolet light emission and subsequent pause. A total time of the ultraviolet light emission in the repeated ultraviolet light emission operations of twice or more may be set to be the time T1. In this case, time of the ultraviolet light emission may be between 10 milliseconds (msec) and 1000 milliseconds, and time of the pause may be between 10 milliseconds and 10 seconds (sec).

The time of the pause allows the longer ultraviolet light irradiation period until the ultraviolet light irradiation time reaches the predetermined time T1, thereby increasing the chance of radiating ultraviolet light when, for example, bacteria or viruses spread and fly at the time of splash and/or defecation.

The ultraviolet light irradiation unit may include a light-emitting diode (LED) or a laser diode (LD) that radiates the ultraviolet light. These devices can repeat the ultraviolet light emission and the pause with high speed by means of power control.

In the above-described inactivation device, the ultraviolet light irradiation unit may include a KrCl excimer lamp that radiates the ultraviolet light having a center wavelength of 222 nm. This configuration suppresses an adversely effect on the human body due to the ultraviolet light irradiation.

In the above-described inactivation device, ultraviolet light contained in the light radiated from the ultraviolet light irradiation unit may only have a wavelength band ranging from 190 nm to 235 nm. This configuration appropriately suppress an adverse effect on the human body due to ultraviolet light irradiation.

A method according to another aspect of the present invention is directed to an inactivation method of inactivating microorganisms and/or viruses harmful to a human body. The method includes: detecting presence of a human in an enclosed space that the human can enter and leave, with a sensor; and controlling an ultraviolet light irradiation unit that irradiates an enclosed space with light containing ultraviolet light having a wavelength suitable for inactivating microorganisms and/or viruses harmful to the human body to perform irradiation and non-irradiation with the light, and irradiating a space including the human with the light for a predetermined time in accordance with the wavelength of the ultraviolet light contained in the light during a period in which the human is determined to be present in the enclosed space.

Hence, intentionally irradiating a human with light containing ultraviolet light for the predetermined time is capable of inactivating at least either harmful microorganisms or viruses present on the surface of human body (surface of skin or clothes). Thus, it is possible to prevent the harmful microorganisms or viruses from spreading into the enclosed space from the human. In addition, it is possible to prevent a human leaving the enclosed space from spreading the harmful microorganisms or viruses outside the enclosed space. Therefore, the irradiation can avoid or reduce the expansion of areas to be decontaminated in a facility, i.e., it is possible to efficiently decontaminate the facility.

The time of irradiating human with ultraviolet light is determined in accordance with the wavelength of the ultraviolet light. The extent to which ultraviolet irradiation adversely affects to the human body depends on the wavelength of the ultraviolet light. Therefore, irradiating the human with ultraviolet light for a predetermined time in accordance with the wavelength of the ultraviolet light is capable of performing the efficient decontamination without adversely affecting to human body.

Advantageous Effects of Invention

The present invention is capable of efficiently inactivating harmful microorganisms or viruses by intentionally irradiating a human with light containing ultraviolet light for a predetermined time.

These and other objects, aspects and advantageous effects of the present invention can be understood by those skilled in the art from the following mode of carrying out the invention (detailed description of the invention) by referring to the accompanying drawings and the claims.

BRIEF DESCRIPTION OF DRAWINGS

FIG. 1 is a view schematically showing an exemplary configuration of an inactivation system according to an embodiment of the present invention.
FIG. 2 is a flowchart describing an operation of a first embodiment.
FIG. 3 is a timing chart describing an operation of the first embodiment.
FIG. 4 is a flowchart describing an operation of a modification to the first embodiment.
FIG. 5 is a timing chart describing an operation of the modification to the first embodiment.
FIG. 6 is a flowchart describing an operation of a second embodiment.
FIG. 7 is a timing chart describing an operation of the second embodiment.
FIG. 8 is a flowchart describing an operation of a modification to the second embodiment.
FIG. 9 is a timing chart describing an operation of the modification to the second embodiment.
FIG. 10 is a timing chart describing an operation of the modification to the second embodiment.

DESCRIPTION OF EMBODIMENTS

Hereinafter, embodiments of the present invention will be described with reference to the drawings.

(First Embodiment)

In the present embodiment, an inactivation system will be described that inactivates harmful microorganisms and viruses by irradiating the harmful microorganism and viruses with ultraviolet light, in particular, in confined spaces of facilities where people frequently gather (enclosed spaces such as hospital rooms, restrooms, and elevators). The inactivation system of the present embodiment inactivates harmful microorganisms and viruses by intentionally irradiating living organisms, such as humans (patients) and animals, with ultraviolet light for a predetermined time, which has been conventionally avoided for safety reason.

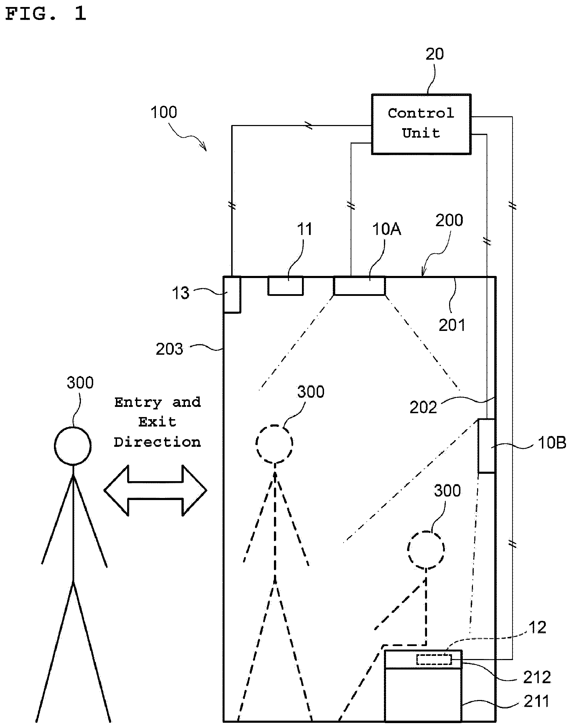

FIG. 1 is a drawing schematically describing an example of a configuration of an inactivation system of the present embodiment. In the present embodiment, an example of an inactivation system that inactivates harmful microorganisms and viruses present in a restroom stall will be described.

The inactivation system includes an inactivation device 100. The inactivation device 100 includes at least one of ultraviolet light irradiation units (UV irradiation units) 10A and 10B that radiate ultraviolet light to an enclosed space (a restroom stall) 200. It is noted that the wavelength band of the ultraviolet light radiated from each of the UV irradiation units 10A, 10B is, for example, 200 nm to 320 nm.

The UV irradiation unit 10A is provided on a ceiling 201 in the restroom stall 200. It is noted that the UV irradiation unit 10A may be provided on the upper part in the restroom stall 200, for example, on the upper area of the wall 202 in the restroom stall 200.

The UV irradiation unit 10A radiates ultraviolet light in a downward direction and the ultraviolet light is directed to a space in the restroom stall 200, the wall 202, a floor and so on. The ultraviolet light radiated from the UV irradiation unit 10A hits a human (for example, patient) 300 from above the human 300 when the human 300 enters the restroom stall 200.

The UV irradiation unit 10B is provided on the wall 202 in the restroom stall 200. The UV irradiation unit 10B mainly radiates ultraviolet light in a downward direction from its mounting position. This UV irradiation unit 10B is mounted at a position such that its ultraviolet light is emitted to the human 300 who takes a predetermined posture at a predetermined position in the restroom stall 200.

Specifically, the UV irradiation unit 10B is mounted on the wall 202 of the restroom stall 200 on the assumption that the ultraviolet light from the UV irradiation unit 10B is directed to the human 300 when the human takes a posture of sitting on a toilet bowl 211. More specifically, the UV irradiation unit 10B is mounted on the wall 202 facing the back of the head of the human 300 when the human sits on the toilet bowl 211.

Hence, mounting the UV irradiation unit 10B in the above-described manner allows the ultraviolet light radiated from the UV irradiation unit 10B to be emitted toward the human 200 from a position upward of the back of the head of the human 200 sitting on the toilet bowl 211, thereby preventing the ultraviolet light from being directly emitted to the eyes of the human 300.

The inactivation device 100 may include at least one of the sensors such as a motion sensor 11, a pressure sensor 12, and a door sensor 13 to detect the presence of the human 300 in the restroom stall 200.

The motion sensor 11 and the door sensor 13 are sensors that detect the entry and exit of a person to and from the restroom stall 200.

The pressure sensor 12 is a sensor that detects the presence of a human at a predetermined position in the restroom stall 200.

The motion sensor 11 is mounted on the ceiling 201, for example, as shown in FIG. 1, and detects the presence or absence of the human 300 in the space of the restroom stall 200. The pressure sensor 12 is mounted in a toilet seat 212, for example, as shown in FIG. 1, and detects whether or not the human 300 sits on the toilet seat 212 provided on the toilet bowl 211.

The door sensor 13 is mounted on the door 203, for example, as shown in FIG. 1, and detects the opening and closing of the door 203 of the restroom stall 200.

Furthermore, the inactivation device 100 includes a control unit 20. The control unit 20 receives detection signals from each of the sensors 11 to 13 and controls the irradiation and non-irradiation with the ultraviolet light from the UV irradiation units 10A and 10B based on the detection signals.

Specifically, the control unit 20 controls at least one of the UV irradiation units 10A and 10B to irradiate the interior of the restroom stall 200 including the human 300 with the ultraviolet light for a predetermined time in accordance with the wavelength of the ultraviolet light while the human 300 is determined to be present in the restroom stall 200 based on at least one of the detection signals from the sensors 11 to 13.

In the present embodiment, is described the case that the control unit 20 controls the irradiation with ultraviolet light radiated from the UV irradiation unit 10A based on the detection signal from the motion sensor 11. Furthermore, in the present embodiment, if the human 300 is absent in the restroom stall 200, ultraviolet light principally continues to be emitted in the restroom stall 200 from the UV irradiation unit 10A.

Hereinafter the operation of the inactivation device 100 according to the present embodiment will be described.

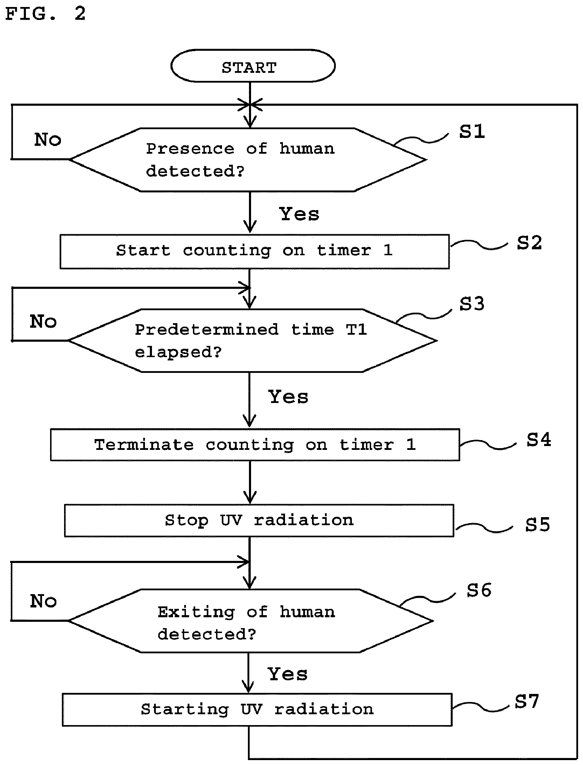

FIG. 2 is a flowchart describing an operation of the inactivation device 100 according to the present embodiment.

In Step S1, the control unit 20 determines whether or not the presence of the human 300 is detected in the restroom stall 200 based on the detection signal from the motion sensor 11. If the control unit 20 determines that presence of the human 300 is not detected, the control unit 20 waits until the presence of the human 300 is detected. Upon detecting the presence of the human 300, the operation proceeds to Step S2.

In Step S2, the control unit 20 causes a timer 1 to start counting as a counter.

Next in Step S3, the control unit 20 determines whether or not a predetermined time T1 has elapsed since the timer 1 start the counting based on the counter value of the timer 1, i.e., the control unit 20 determines whether or not the predetermined time T1 has elapsed since the detection of the presence of the human 300 in the restroom stall 200. If the predetermined time T1 has not yet elapsed, the control 20 waits for the predetermined time T1 to elapse. The control unit 20 proceeds to Step S4 when it determines that the predetermined time T1 has elapsed.

It is noted that the predetermined time T1 is determined in accordance with the wavelength of the ultraviolet light radiated from the UV irradiation unit 10A, and should be set not more than a maximum time that is permitted to be emitted to a living body under safety standards. Details of the predetermined time T1 will be explained later.

In Step S4, the control unit 20 causes the timer 1 to stop counting and resets the counting value of the timer 1.

In Step S5, the control unit 20 causes the UV irradiation unit 10A to stop emitting the ultraviolet light.

In Step S6, the control unit 20 determines whether or not the human 300 has exited from the restroom stall 200 based on the detection signal from the motion sensor 11. The control unit 20 waits until it determines the exiting of the human 300. The control unit 20 proceeds to Step S7 when it determines that the human 300 has exited.

In Step S7, the control unit 20 causes the UV irradiation unit 10A to start emitting ultraviolet light and returns to Step S1.

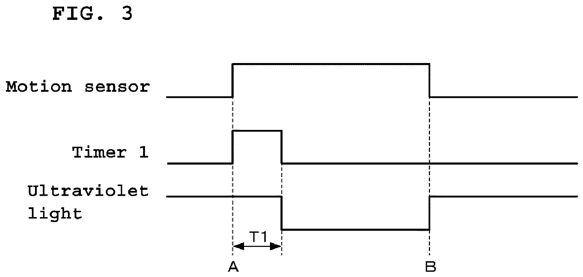

FIG. 3 is a timing chart describing an operation of the inactivation device 100 of the present embodiment.

Ultraviolet light from the UV irradiation unit 10A continues to be emitted in the restroom stall 200 before the human 300 enters the restroom stall 200, i.e., during the absence of the human 300 in the restroom stall 200.

As the human 300 enters the restroom stall 200 at the time A, the motion sensor 11 detects the presence of the human 300, and the timer 1 starts counting. After the predetermined time T1 has elapsed from the time A, the emission of ultraviolet light in the restroom stall 200 and to the human 300 is stopped.

Accordingly, even after the human 300 enters the restroom stall 200, ultraviolet light from UV irradiation unit 10A continues to be emitted in the restroom stall 200, and to the human 300 inside the restroom stall 200 until the predetermined time T1 has elapsed.

When the human 300 exits the restroom stall 200 at the time B, the motion sensor 11 detects the exiting of the human 300, and the emission of the ultraviolet light (irradiation with the ultraviolet light) is resumed in the restroom stall 200.

The irradiation time (predetermined time T1) for irradiating the human 300 with the ultraviolet light will now be explained.

Ultraviolet light having a wavelength range of 200 nm to 320 nm radiated from each of the UV irradiation units 10A and 10B contains ultraviolet light that adversely affects human bodies. For example, ultraviolet irradiation in the above-mentioned wavelength range can induce erythema and cancer due to DNA damage in the skin, and cause eye damage (eye pain, redness, inflammation of the cornea, etc.).

It should be noted, however, that ultraviolet irradiation in the above-mentioned wavelength range does not adversely affect organisms unless integral light intensity (dose amount) to a living body as a subject to be irradiated exceeds a predetermined quantity. The present inventers focus on this point and set irradiation time (predetermined time T1) to intentionally irradiate humans with ultraviolet light.

An amount of ultraviolet light exposure to a human using an enclosed space in one day (restroom stall) is denoted as D ($mJ/cm^2$). The amount of ultraviolet exposure in one day "D" is expressed by the following equation.

$$D \ (mJ/cm^2) = W \ (mW/cm^2) \times N \times T1 \ (sec) \cdot \cdot \cdot (1)$$

where W ($mW/cm^2$) is the irradiance of the ultraviolet light emitted on the surface of the human body, N is the number of times the human enters the enclosed space (restroom stall) in one day, and T1 is the ultraviolet light irradiation time during a single stay in the restroom stall.

When $D_{max}$ ($mJ/cm^2$) denotes the maximum amount of allowable ultraviolet light exposure to the human body using the enclosed space (restroom stall) in one day, $D_{max} \geq D$ is sufficient to prevent adverse effect on humans caused by ultraviolet light irradiation.

Namely, the ultraviolet light irradiation time per stay in the restroom stall T1 is expressed by the following formula.

$$T1 \leqq D_{max} / (W \times N) \cdot \cdot \cdot (2)$$

As an example, a low-pressure mercury lamp that emits ultraviolet light having a wavelength of 253.7 nm is used for the ultraviolet light source. The maximum amount of allowable exposure of the ultraviolet light having a wavelength of 253.7 nm is expressed as $D_{max} = 6$ (mJ/cm$^2$) due to the safety standard. This value is determined by the ACGIH (American Conference of Governmental Industrial Hygienists).

The ultraviolet light irradiation time during a single stay in the restroom stall T1 is calculated to be 30 seconds or less by using the equation (2), where the irradiance of the ultraviolet light directed onto the surface of the human body is 0.022 (mW/cm$^2$) and the number of times N the human uses the restroom stall provided in a hospital room (number of times the human enters the restroom stall) per day is 10.

In other words, in the case in which the ultraviolet light source provided in the UV irradiation unit 10A is a low-pressure mercury lamp that radiates ultraviolet light having a wavelength of 253.7 nm, ultraviolet light irradiation does not adversely affect the human 300 as long as the predetermined time T1 set in FIG. 2 and FIG. 3 is 30 seconds or less. Hence, in this case, the predetermined time T1 is set to be, for example, 30 seconds, which is a maximum time.

It is noted that the irradiance of the ultraviolet light emitted onto the surface of the human body is determined using the following considerations: the head (top) of the human 300 standing in the enclosed space (restroom stall) 200 is considered to be the surface to which the ultraviolet light is emitted, and the distance from the ceiling 201 of the enclosed space (restroom stall) 200 to the head of the human 300 standing on the floor is considered to be the ultraviolet light travelling distance.

The number of times N a human enters the enclosed space (restroom stall) refers to the number of times a human uses the restroom stall provided in a hospital room, thus N = 10. In the case of the restroom stall that is provided in a reception area or a waiting room for outpatients, however, the number of times N' that each of a plurality of humans waiting in a waiting room uses the restroom stall per day is considered to be smaller than the number of times N a patient uses a restroom stall provided in a hospital room. Hence, N' = 2 or 3 may be used to set the ultraviolet light irradiation time T1.

It is preferable that the number of times human enters the enclosed space per day N be set to a larger number for a safety side.

In addition, low-pressure mercury lamps do not immediately light up when power is supplied; it takes certain amount of time to light up. Hence, a low-pressure mercury lamp used as an ultraviolet light source cannot repeatedly perform irradiation and non-irradiation with the ultraviolet light at relatively short intervals using the power control. In this case, shutters for light shielding may be provided and controlled to open and close while keeping a low-pressure mercury lamp in a lit state such that emission and non-emission of ultraviolet light is controlled.

The example of the ultraviolet light source is a KrCl excimer lamp that radiates ultraviolet light having a center wavelength of 222 nm.

Excimer lamps light up immediately after its power is supplied. Unlike low-pressure mercury lamps used for the light source, no shutter for shielding the light needs to be provided. Hence, controlling the power to the excimer lamps enables the repeated irradiation and non-irradiation with ultraviolet light at relatively short intervals.

The ultraviolet light having a center wavelength of 222 nm sterilizes germs including bacteria; however, it has less adverse effects on human cells.

UV radiation ray having shorter wavelengths penetrates less. For example, UV radiation ray having short wavelengths such as approximately 200 nm is transmitted through water very efficiently; however, it is highly absorbed by the outer portion of human cells (cytoplasm) and may not have enough energy to reach cell nucleus, which contains radiation-sensitive DNA. Hence, the above-mentioned UV radiation ray having the short wavelengths has less adverse effects to human cells, namely, to humans.

In contrast, bacteria is typically much smaller in physical size than human cells. Specifically, typical bacteria cell has a diameter of below 1 micrometer, whereas the human cells typically have a diameter of approximately 10 to 30 micrometers depending upon the kind and location of the human cell.

Hence, UV radiation having short wavelength can readily penetrate bacteria and sterilize them.

The current safety standard permits that the daily maximum amount of allowable exposure of ultraviolet light having a wavelength of 222 nm is $D_{max} = 21$ (mJ/cm$^2$). This value is larger than that of ultraviolet light having a wavelength of 253.7 nm. In other words, from the standpoint of the safety standard, the ultraviolet light having a wavelength of 222 nm has less adverse effects on humans than the ultraviolet light having a wavelength of 253.7 nm.

In the case of adopting KrCl excimer lamps that emit ultraviolet light having a center wavelength of 222 nm, the ultraviolet light irradiation time per stay in the restroom stall T1 is calculated to be 95 seconds or less using the equation (2), where the irradiance of the ultraviolet light emitted onto the surface of the human body is 0.022 (mW/cm$^2$) and the number of times N the human uses the restroom stall provided in a hospital room (number of times entering the restroom stall) per day is 10, which is similar to the case of adopting low-pressure mercury lamps that radiate ultraviolet light having a wavelength of 253.7 nm.

Thus, in the case that the ultraviolet light source provided in the UV irradiation unit 10A is KrCl excimer lamps that radiate ultraviolet light having a center wavelength of 222 nm, the predetermined time T1 set in FIG. 2 and FIG. 3 is determined to be, for example, a maximum time of 95 seconds.

KrCl excimer lamps radiate ultraviolet light having a center wavelength of 222 nm; however, they also slightly radiate light having the other wavelength ranges. Thus, for practical use of KrCl excimer lamps, it is preferable to use a wavelength selection filter that transmits only light having a wavelength band ranging from 190 nm to 235 nm, which has less adverse effect on humans, and blocks light having the other wavelength bands.

The wavelength selection filter can be, for example, an optical filter having a dielectric multilayer with $HfO_2$ and $SiO_2$ layers. Specifically, the optical filter may have a structure in which a dielectric multilayer film consisting of alternating layers of $HfO_2$ and $SiO_2$ is formed on one surface of a substrate made of synthetic quartz (silica) glass, and an AR coating consisting of $HfO_2$ and $SiO_2$ layers is applied on the other surface of the substrate. The dielectric multilayer has, for example, $HfO_2$ layers having a thickness of approximately 240 nm and $SiO_2$ layers having a thickness of approximately 1460 nm, and consists of 33 layers (sum of the $HfO_2$ layers and the $SiO_2$ layers) in total.

The wavelength selection filter can also be, for example, an optical filter having a dielectric multilayer with $SiO_2$ and $Al_2O_3$ layers.

However, when the optical filter with a dielectric multilayer made of $HfO_2$ and $SiO_2$ layers is used as a wavelength selection filter, the total number of layers is reduced as compared to the case where an optical filter with dielectric multilayer made of $SiO_2$ and $Al_2O_3$ layers is used. Therefore, the transmittance of ultraviolet light at an incident angle of 0° increases, ensuring the light intensity of ultraviolet light in the desired wavelength band of 190 nm to 235 nm. In addition, reducing the total number of layers decreases the cost for fabrication correspondingly.

As described above, the inactivation device 100 of the present embodiment includes the ultraviolet light irradiation unit (UV irradiation unit) 10A that radiates light containing ultraviolet light having a wavelength of inactivating harmful bacteria and/or viruses to a human body to the enclosed space (the restroom stall 200) where a human can enter and leave. The inactivation device 100 also includes the motion sensor 11 that detects the presence and absence of the human in the restroom stall 200 as a sensor to detect the presence of the human in the restroom stall 200. The control unit 20 controls the UV irradiation unit 10A to irradiate the restroom stall 200 including the human with the ultraviolet light for a predetermined time (T1) in accordance with the wavelength of the ultraviolet light radiated from the UV irradiation unit 10A while the human is determined to be present in the restroom stall 200 based on the detection signal from the motion sensor 11.

In this way, intentionally irradiating a human with light containing ultraviolet light for the predetermined time T1 is capable of inactivating at least one harmful microorganisms or viruses present on the surface of human body (skin or surface of clothes). Hence, this irradiation prevents the harmful microorganisms and/or viruses attached to humans from spreading into an enclosed space (the restroom stall 200) or reduces the spreading of the harmful microorganisms and/or viruses from humans in the enclosed space.

In addition, the human leaves the enclosed space (the restroom stall 200) after being irradiated with ultraviolet light. Thus, the harmful microorganisms and/or viruses attached to skin or the surface of clothes has been decreased or eliminated. This prevents the human leaving the enclosed space (the restroom stall 200) from spreading the harmful microorganisms and/or viruses outside the enclosed space or reduces the spreading of harmful microorganisms and/or viruses from the human outside the enclosed space. Therefore, it is possible to prevent or reduce expansion of the area to be decontaminated in the facility, and achieve efficient decontamination of the facility.

Moreover, the predetermined time T1 of irradiating a human with ultraviolet light is determined in accordance with the wavelength of the ultraviolet light. Because the extent to which ultraviolet irradiation adversely affects to a human body depends on the wavelength of the ultraviolet light, setting the predetermined time T1 in accordance with the wavelength of the ultraviolet light allows ultraviolet light having a wavelength suitable for the decontamination to be emitted to the human within a range of amount of light that does not adversely affect to the human body.

Specifically, the predetermined time T1 is determined to satisfy the equation (2). Thus, the ultraviolet light irradiation time is determined in accordance with the wavelength of the emitted ultraviolet light based on the safety standard, and it is, therefore, possible to appropriately suppress the adverse effect of the ultraviolet light on the human body.

The control unit 20 may acquire the information on the wavelength of ultraviolet light emitted from the UV irradiation unit 10A, set the predetermined time T1 based on the acquired information together with the safety standard, and control the light emission and no light emission (irradiation and non-irradiation) of the UV irradiation unit 10A. In other words, the inactivation device may be configured such that the predetermined time T1 is variable and set in accordance with the light source used.

In addition, in the present embodiment, the UV irradiation unit 10A can be controlled to irradiate the space including a human with ultraviolet light for the predetermined time T1 after the motion sensor 11 detects the human entering the restroom stall 200. In this way, it is possible to emit the ultraviolet light to the human immediately after the human enters the restroom stall 200. Therefore, it is possible to suppress the human from spreading harmful bacteria and/or viruses into the restroom stall 200 in a more efficient manner.

Moreover, in the present embodiment, the UV irradiation unit 10A can be controlled to radiate ultraviolet light in the restroom stall 200 where no human is present at the time when the motion sensor 11 determines that no human is present in the restroom stall 200 (a human has left the restroom stall 200).

In this way, irradiation with ultraviolet light in the restroom stall 200 where no human is present is capable of inactivating at least part of harmful microorganisms and viruses already present inside the restroom stall 200, the harmful microorganisms and viruses spreading inside the restroom stall 200 as a human enters the restroom stall, and the harmful microorganisms and viruses floating in the air introduced into the restroom stall 200 as the human enters the restroom stall. Moreover, ultraviolet light is emitted continuously in the restroom stall 200 when no human is present in the restroom stall 200, enhancing the above-described inactivation.

The UV irradiation unit 10A may be mounted at a position that allows light to proceed downward from the upper part of the restroom stall 200. Specifically, the UV irradiation unit 10A may be mounted on the ceiling 201 in the restroom stall 200. Thus, the UV irradiation unit 10A is able to emit light containing ultraviolet light to the entire interior of the restroom stall 200. Therefore, this configuration appropriately inactivates harmful microorganisms and viruses attached to, for example, the wall 202, the door 203, and the floor of the restroom stall 200.

In the present embodiment, the motion sensor 11 for detecting the presence or absence of human in the restroom stall 200 is described as a sensor for detecting the human entering or exiting the restroom stall 200; however, any sensor that detects the human entering the restroom stall 200 and the human leaving the restroom stall 200 can be used.

(Modification to the first embodiment)

The first embodiment has described the case in which the UV irradiation unit 10A continuously radiates ultraviolet light in the restroom stall 200 when no human 300 is present in the restroom stall 200. However, ultraviolet light may be emitted during a predetermined time T2 in the restroom stall 200 when no human is present in the restroom stall 200.

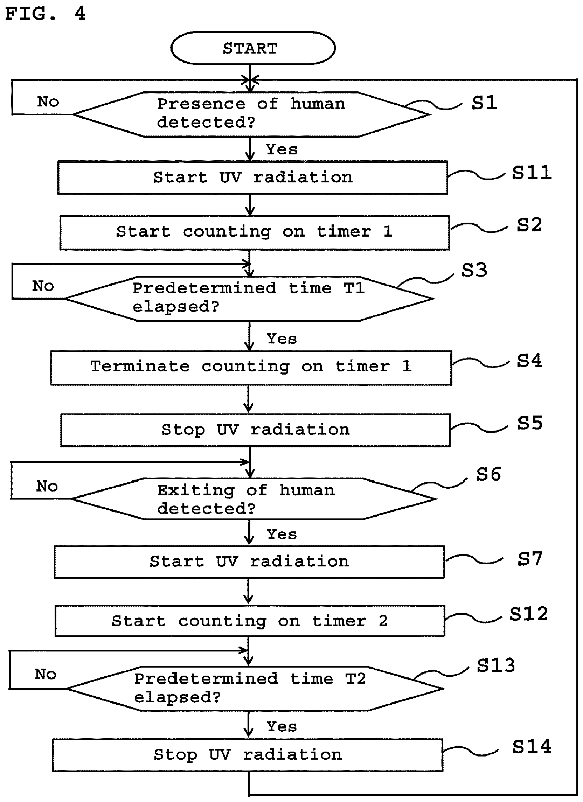

FIG. 4 is a flowchart describing an operation of the inactivation device 100 according to the present modification. In FIG. 4, the processes that are common to those in FIG. 2 are denoted with the identical step numbers; hereinafter, the processes different from those of FIG. 2 will be mainly described.

When the control unit 20 detects the presence of the human 300 in the restroom stall 200 at Step S1, it proceeds to Step S11 to start ultraviolet light emission from the UV irradiation unit 10A, and then proceeds to the step S2.

In Step S7, the control unit 20 causes the UV irradiation unit 10A to start ultraviolet light emission. Subsequently, the control unit 20 proceeds to Step S12 and causes a timer 2, which is a counter, to start counting.

Next, in Step S13, the control unit 20 determines whether or not the predetermined time T2 has elapsed since the start of the counting of the timer 2 based on the count value of the timer 2, i.e., whether or not the predetermined time T2 has elapsed since the human 300 exits the restroom stall 200. In the case that the predetermined time T2 has not yet elapsed, the control unit 20 waits until the predetermined time T2 has elapsed, and proceeds to Step S14 when the predetermined time T2 is determined to have elapsed.

It is noted that the predetermined time T2 is set to a time sufficient to inactivate at least part of harmful microorganisms and viruses present in the restroom stall 200 where the human 300 has exited.

In Step S14, the control unit 20 stops ultraviolet light emission from the UV irradiation unit 10A and returns to Step S1.

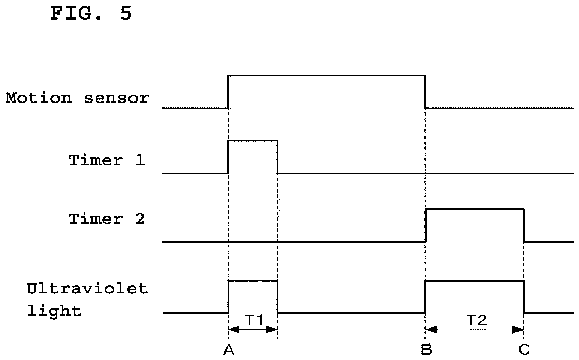

FIG. 5 is a timing chart describing an operation of the inactivation device 100 according to the modification.

In this modification, it is assumed that the radiation of ultraviolet light from the UV irradiation unit 10A to the restroom stall 200 has been stopped before the human 300 enters the restroom stall 200.

When the human 300 enters the restroom stall 200 at the time A, the motion sensor 11 detects the presence of the human 300, the timer 1 starts counting and ultraviolet light is emitted in the restroom stall 200 and to the human 300. The radiation of ultraviolet light in the restroom stall 200 and to the human 300 is stopped after the predetermined time T1 has elapsed since the time A.

In this way, even in the case in which the UV irradiation unit 10A stops emitting ultraviolet light before the human 300 enters the restroom stall 200, the UV irradiation unit 10A starts emitting ultraviolet light once the human 300 enters the restroom stall 200. The UV irradiation unit 10A keeps irradiating the human 300 in the restroom stall 200 with ultraviolet light during a period from the time of starting the ultraviolet light emission until the predetermined time T1 has elapsed.

After that, when the human 300 exits the restroom stall 200 at the time B, the motion sensor 11 detects the exiting of the human 300, the timer 2 starts counting and the emission of ultraviolet light to the interior of the restroom stall 200 is restarted.

The emission of ultraviolet light to the interior of the restroom stall 200 is stopped at the time C when the predetermined time T2 has elapsed since the time B, i.e., since the human 300 left the restroom stall 200.

In this way, in the case in which the motion sensor 11 determines that no human is present in the restroom stall 200, ultraviolet light may be emitted from the UV irradiation unit 10A to the restroom stall 200 where no human is present for a certain period (predetermined time T2), and then the emission of ultraviolet light from the UV irradiation unit 10A may be stopped. By emitting ultraviolet light to the interior of the restroom stall 200 where no human is present for a certain period, it is possible to set a pause time of the ultraviolet light source provided in the UV irradiation unit 10A, thereby extending a service life of the ultraviolet light source.

(Second Embodiment)

Hereinafter, the second embodiment of the present invention will be described.

In the first embodiment described above, described is the case in which the motion sensor 11 detects the human 300 entering the enclosed space (the restroom stall 200) and the irradiation with ultraviolet light from the UV irradiation unit 10A is controlled based on the detection signal of the motion sensor 11. In the second embodiment, described is the case in which a pressure sensor 12 detects the state that the human 300 sits on the toilet seat 212 in the restroom stall 200 and the irradiation with ultraviolet is controlled based on the detection signal of the pressure sensor 12.

In the present embodiment, ultraviolet light emission generally continues in the restroom stall 200 when no human 300 is present in the restroom stall 200.

The ultraviolet light emission is to be carried out using the UV irradiation unit 10B.

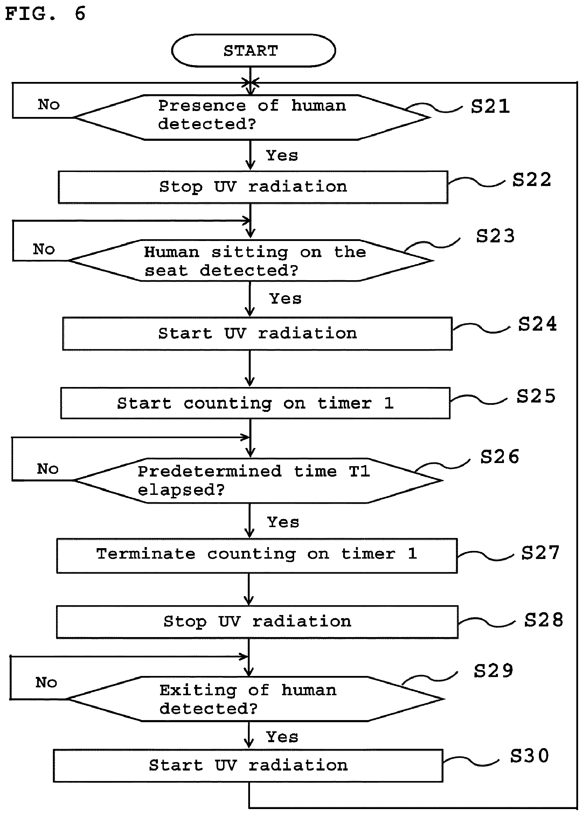

FIG. 6 is a flowchart describing an operation of the inactivation device 100 according to the present embodiment.

In Step S21, the control unit 20 determines whether or not the presence of the human 300 is detected in the restroom stall 200 based on the detection signal of the motion sensor 11. In the case of determining that the presence of human 300 is not detected, the control unit 20 waits until the presence of the human 300 is detected. The control unit 20 proceeds to Step S22 when the presence of the human 300 is detected.

In Step S22, the control unit 20 stops ultraviolet light emission from the UV irradiation unit 10B and proceeds to Step S23.

In Step S23, the control unit 20 determines whether or not the human 300 sits on the toilet seat 212 based on the detection signal of the pressure sensor 12. In the case of determining that no human 300 sitting on the seat is detected, the control unit 20 waits until the human 300 sitting on the seat is detected. The control unit 20 proceeds to Step S24 when the human 300 sitting on the seat is detected.

In Step S24, the control unit 20 starts ultraviolet light emission from the UV irradiation unit 10B and proceeds to Step S25.

In Step S25, the control unit 20 starts the counting of the timer 1, which is a counter.

Next, in Step S26, the control unit 20 determines whether or not the predetermined time T1 has elapsed since the start of the counting of the timer 1 based on the counter value of the timer 1, i.e., whether or not the predetermined time T1 has elapsed since the detection of the human 300 sitting on the toilet seat 212. In the case that the predetermined time T1 has not yet elapsed, the control unit 20 waits for the predetermined time T1 to elapse, and then proceeds to Step S27 upon the determination that the predetermined time T1 has elapsed.

It is noted that the predetermined time T1 is determined according to the wavelength of the ultraviolet light radiated from the UV irradiation unit 10B, and is set to be not more than a maximum allowable time for which a living body is irradiated under the safety standards. The predetermined time T1 may be, for example, a value similar to that of the first embodiment.

The UV irradiation unit 10B is mounted on the wall 202 of the restroom stall 200 on the assumption that its ultraviolet light is emitted from above the back of the head of the human 300 when the human is in a posture of sitting on the toilet bowl 211. Thus, the irradiance on the surface (head) of the human 300 in this case has a value similar to the irradiance on the surface of the standing human 300 using the UV irradiation unit 10A in the first embodiment. In other words, the irradiance of the ultraviolet light emitted on the surface of the human body can be 0.092 (mW/cm$^2$).

In Step S27, the control unit 20 terminates the counting of the timer 1 and resets the count value of the timer 1.

In Step S28, the control unit 20 stops ultraviolet light emission from the UV irradiation unit 10B.

In Step S29, the control unit 20 determines whether or not the human 300 exits the restroom stall 200 based on the detection signal of the motion sensor 11. In the case of determining that the human 300 does not exit, the control unit 20 waits. The control unit 20 proceeds to Step S30 upon the determination that the human 300 has exited.

In Step S30, the control unit 20 causes the UV irradiation unit 10B to start emitting ultraviolet light and returns to Step S21.

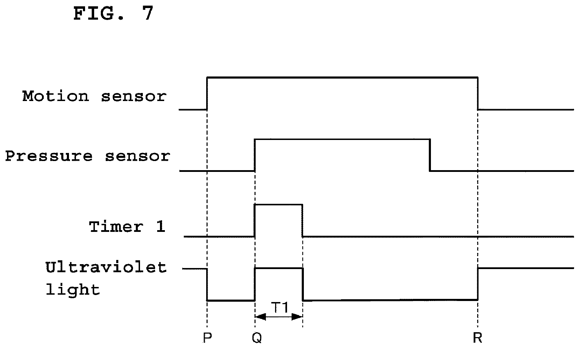

FIG. 7 is a timing chart describing an operation of the inactivation device 100 of the present embodiment. Ultraviolet light emission from the UV irradiation unit 10B continues in the restroom stall 200 before the human 300 enters the restroom stall 200, i.e., during the absence of the human 300 in the restroom stall 200.

When the human 300 enters the restroom stall 200 in this state at the time P, the motion sensor 11 detects the presence of the human 300, and the irradiation with ultraviolet light in the restroom stall 200 is stopped.

After that, when the human 300 sits on the toilet seat 212 in the restroom stall 200 at the time Q, the pressure sensor 12 detects the human 300 sitting on the seat, the timer 1 starts counting and the irradiation with ultraviolet light is started in the restroom stall 200. The emission of ultraviolet light in the restroom stall 200 and to the human 300 is stopped after the predetermined time T1 has elapsed since the time Q.

In this way, when the human 300 enters the restroom stall 200, the irradiation with ultraviolet light from the UV irradiation unit 10B is temporarily stopped; when the human 300 sits on the toilet seat 212, the UV irradiation unit 10B radiates ultraviolet light in the restroom stall 200 such that the human 300 is irradiated with ultraviolet light for the predetermined time T1.

After that, when the human 300 exits the restroom stall 200 at the time R, the motion sensor 11 detects the exiting of the human 300, and the irradiation with ultraviolet light is restarted in the restroom stall 200.

As described above, in the present embodiment, the control unit 20 causes the UV irradiation unit 10B to irradiate the space including the human with ultraviolet light for the predetermined time (T1) upon detecting the human sitting on the toilet seat 212 based on the detection signal of the pressure sensor 12 during the period in which the human is determined to be present in the restroom stall 200 based on the detection signal of the motion sensor 11.

Hence, by irradiating the human present in the predetermined position in the enclosed space with light containing ultraviolet light, it is possible to direct ultraviolet light effectively to the intended location on the surface of a human body. Incidentally, movements of the human sitting on the toilet seat 212 are relatively small in the restroom stall 200. Thus, irradiating the human sitting on the toilet seat 212 with ultraviolet light is capable of effectively inactivating harmful microorganisms and viruses on the surface of the human body (skin and clothing).

Moreover, the control unit 20 causes the UV irradiation unit 10B to stop emitting ultraviolet light when the motion sensor 11 detects the human entering the restroom stall 200, and causes the UV irradiation unit 10B to start irradiating the space including the human with ultraviolet light for the predetermined time (T1) after the pressure sensor 12 detects the human sitting on the toilet seat 212.

Hence, in the case in which ultraviolet light is emitted in the restroom stall 200 before the human enters there, the emission of ultraviolet light can temporarily be stopped when the human enters the restroom stall 200, and the emission of ultraviolet light can be started when the human sits on the toilet seat 212 and continue for the predetermined time (T1). Therefore, this operation suitably enables the emission of ultraviolet light in the restroom stall 200 during absence of the human and the emission of ultraviolet light to the human entering the restroom stall 200.

Moreover, since the UV irradiation unit 10B is mounted on the wall 202 in the restroom stall 200 on the assumption that its ultraviolet light is directed to the human 300 when the human 300 is in a posture of sitting on the toilet bowl 211, the emission of ultraviolet light using the UV irradiation unit 10B increases the dose amount on the floor compared with that using the UV irradiation unit 10A. Therefore, the UV irradiation unit 10B effectively inactivates harmful microorganisms and viruses attached to the floor.

In addition, the UV irradiation unit 10B is mounted at a position where ultraviolet light is emitted to the back of the head of the human 300 when the human 300 is in a posture of sitting on the toilet bowl 211. Hence, this configuration prevents the human's eyes from directly being exposed to ultraviolet light radiated from the UV irradiation unit 10B. Thus, it is possible to suppress the occurrence of eye damages (e.g., eye pain, hyperemia, and corneal inflammation).

Although the present embodiment has described the case in which the irradiation with ultraviolet light is carried out using the UV irradiation unit 10B, the UV irradiation unit 10A mounted on the ceiling 201 in the restroom stall 200 can be used in the present embodiment.

In the case of using the UV irradiation unit 10A, the irradiance of the ultraviolet light on the surface of the human 300 sitting on the toilet seat 212 is small compared with that on the surface of the human 300 standing on the floor, and is, for example, 0.010 (mW/cm$^2$).

Hence, in the case in which a light source provided in the UV irradiation unit 10A is a low-pressure mercury lamp, the ultraviolet light irradiation time during a single stay in the restroom stall (the predetermined time T1) is calculated to be 60 seconds by using the equation (2), where the number of times N using the restroom stall provided in a hospital (number of times entering the restroom stall) per day is 10.

In the case in which a light source provided in the UV irradiation unit 10A is a KrCl excimer lamp, the ultraviolet light irradiation time during a single stay in the restroom stall (the predetermined time T1) is calculated to be 210 seconds by using the equation (2), where the number of times N using the restroom stall provided in a hospital (number of times entering the restroom stall) per day is 10.

Hence, the UV irradiation unit 10A has a longer ultraviolet light irradiation time (the predetermined time T1) than the UV irradiation unit 10B.

Although the present embodiment has described the case in which a pressure sensor 12 provided in the toilet seat 212 is used as a sensor to detect a state that the human sits on the toilet seat 212 in the restroom stall 200, any sensor that detects the state that the human sits on the toilet seat 212 can be used.

(Modification 1 to the second embodiment)

The above-described second embodiment has dealt with the case in which the UV irradiation unit 10A continuously radiates ultraviolet light in the restroom stall 200 when no human 300 is present in the restroom stall 200. However, ultraviolet light may be emitted for only a predetermined time T2 in the restroom stall 200 when no human is present in the restroom stall 200.

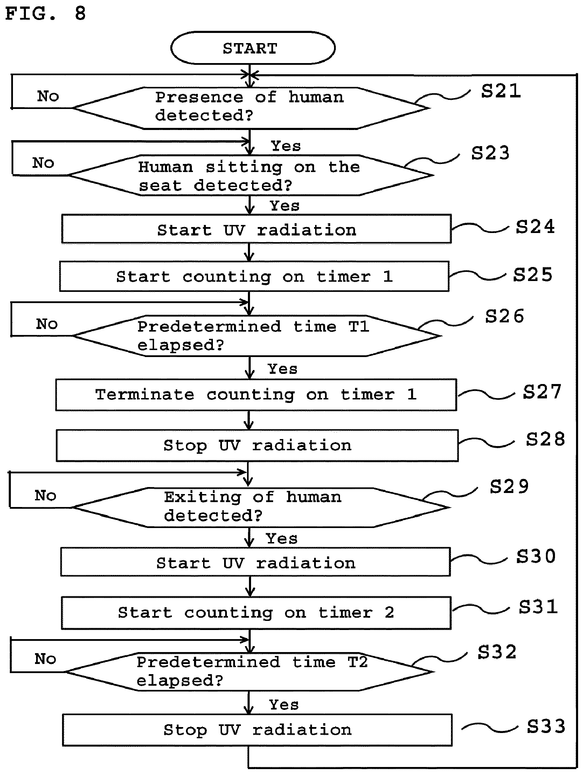

FIG. 8 is a flowchart describing an operation of the inactivation device 100 according to the present modification. In FIG. 8, the processes that are common to those in FIG. 6 are denoted with the identical step numbers; hereinafter, the processes different from those of FIG. 6 will be mainly described.

When the control unit 20 detects the presence of the human 300 in the restroom stall 200 in Step S21, then it proceeds to Step S23.

In addition, the control unit 20 causes the UV irradiation unit 10B to start radiating ultraviolet light in Step S30, and then it proceeds to Step S31 to cause the timer 2, which is a counter, to start counting.

Next, in Step S31, the control unit 20 determines whether or not the predetermined time T2 has elapsed since the start of counting the timer 2 based on the count value of the timer 2, i.e., whether or not the predetermined time T2 has elapsed since the human 300 exits the restroom stall 200. Then, in the case that the predetermined time T2 has not yet elapsed, the control unit 20 waits until the determined time T2 has elapsed. The control unit 20 proceeds to Step S32 when the predetermined time T2 is determined to have elapsed.

It is noted that the predetermined time T2 is set to a time sufficient to inactivate at least part of harmful microorganisms and viruses present in the restroom stall 200 where the human has exited.

In Step S33, the control unit 20 controls the UV irradiation unit 10B to stop emitting ultraviolet light and returns to Step S21.

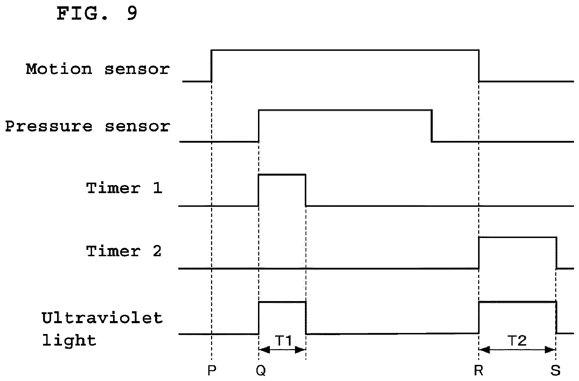

FIG. 9 is a timing chart describing an operation of the inactivation device 100 according to the present modification.

It is assumed here that the irradiation with ultraviolet light from the UV irradiation unit 10B in the restroom stall 200 has been stopped before the human enters the restroom stall 200.

When the human 300 enters the restroom stall 200, the motion sensor 11 detects the presence of the human 300 at the time P After that, when the human 300 sits on the toilet seat 212 at the time Q, the pressure sensor 12 detects the human sitting on the seat. Then, the timer 1 starts counting and ultraviolet light is emitted in the restroom stall 200 and to the human 300. The emission of ultraviolet light in the restroom stall 200 and to the human 300 is stopped after the predetermined time T1 has elapsed since the time Q.

In this way, even in the case in which the UV irradiation unit 10A has stopped the irradiation with ultraviolet light before the human 300 enters the restroom stall 200, the UV irradiation unit 10B starts emitting ultraviolet light once the human 300 enters the restroom stall 200 and sits on the toilet seat 212. The UV irradiation unit 10B emits ultraviolet light to the human 300 in the restroom stall 200 for a period from the time of starting the ultraviolet light emission until the predetermined time T1 has elapsed.

As the human 300 exits the restroom stall 200 at the time R, the motion sensor 11 detects the exiting of the human 300. Then, the timer 2 starts counting and the irradiation with ultraviolet light in the restroom stall 200 is restarted.

The irradiation with ultraviolet light is stopped at the time S, i.e., when the predetermined time T2 has elapsed since the time R that indicates the exiting of the human 300 from the restroom stall 200.

Thus, in the case in which the motion sensor 11 determines that no human is present in the restroom stall 200, ultraviolet light may be emitted from the UV irradiation unit 10B to the restroom stall 200 where no human is present for a certain period (predetermined time T2). After that, the irradiation with ultraviolet lightfrom the UV irradiation unit 10B may be stopped. By emitting ultraviolet light in the restroom stall 200 where no human is present for a certain period, it is possible to set a pause time of the ultraviolet light source provided in the UV irradiation unit 10B, thereby extending a service life of the ultraviolet light source.

(Modification 2 of the second embodiment)

Although the second embodiment has described the case in which the motion sensor 11 detects the presence of the human 300 in the restroom stall 200, the door sensor 13 may be used to detect the human 300 entering or leaving the restroom stall 200.

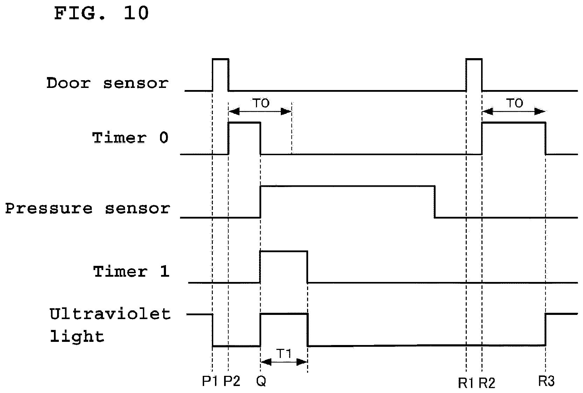

FIG. 10 is a timing chart describing an operation of the inactivation device 100 according to the present modification. The UV irradiation unit 10B continuously emits ultraviolet light in the restroom stall 200 before the human 300 enters the restroom stall 200, i.e., during the absence of the human 300 in the restroom stall 200.

In this state, when the human 300 opens the door 203 to enter the restroom stall 200 at the time P1, the door sensor 13 detects the door 203 opening and the irradiation with ultraviolet light in the restroom stall 200 is stopped. After that, when the human 300 enters the restroom stall 200 and closes the door 203 at the time P2, the door sensor 13 detects the door 203 closed.

Then, the timer 0 starts counting at the time P2. It is noted that the timer 0 is set to terminate the counting at the time when the predetermined time T0 has elapsed since the start of the counting, and reset the count while sending a counting terminate signal to the control unit 20. The timer 0 is also set to be reset by the control unit 20 in the case in which the pressure sensor 12 detects the human 300 sitting on the toilet seat 212 even during the middle of its counting.

The predetermined time T0 is set to a time sufficiently longer than a time for the human 300 to sit on the toilet seat 212 after entering the restroom stall 200.

After that, when the human 300 sits on the toilet seat 212 in the restroom stall 200 at the time Q, the pressure sensor 12 detects the human 300 sitting on the seat, the timer 1 starts counting and ultraviolet light emission starts in the restroom stall 200. At this moment, the timer 0 terminates its counting. The emission of ultraviolet light in the restroom stall 200 and to the human 300 is stopped after the predetermined time T1 has elapsed since the time Q.

In this way, when the human 300 enters the restroom stall 200, the irradiation with ultraviolet light from the UV irradiation unit 10B is temporarily stopped; however, when the human 300 sits on the toilet seat 212, the UV irradiation unit 10B radiates ultraviolet light in the restroom stall 200 and ultraviolet light is directed to the human 300 in the restroom stall 200 during the predetermined time T1.

After that, when the human 300 opens the door 203 to exit the restroom stall 200 at the time R1, the door sensor 13 detects the door 203 opening. Then, when the human 300 exits the restroom stall 200 and the door 203 is closed at the time R2, the door sensor 13 detects the door 203 closed.

At the time R2, the timer 0 starts counting. Since the human 300 has exited the restroom stall 200, the pressure sensor 12 does not detect the human sitting on the toilet seat 212 even when the predetermined time T0 elapses from the time R2. Hence, the irradiation with ultraviolet light in the restroom stall 200 is restarted at the time R3, i.e., when the predetermined time T0 elapses from the time R2.

In this way, even in the case in which the door sensor 13 is used instead of the motion sensor 11, the effect similar to that of the second embodiment described above is achieved if the count of the timer 0 is used. Since the door sensor 13 detects the opening and closing of the door 203, ultraviolet light can be radiated in the enclosed space (the restroom stall 200) with the door 203 closed. This configuration prevents ultraviolet light from being accidentally emitted to objects outside the enclosed space.

Here, described is the case in which the control unit 20 controls the timer 0 to start counting when the door sensor 13 detects the door 203 closed; however, the control unit 20 may control the timer 0 to start counting when the door sensor 13 detects the door 203 closed and, at the same time, the pressure sensor 12 does not detect the human 300 sitting on the seat.

This case makes it possible to have the timer 0 not to start counting in the case in which the door 203 undesirably opens or closes due to reasons including forgotten locking of the door 203 or the defect of the door 203 while the human 300 is sitting on the toilet seat 212.

In other words, in the case in which the control unit 20 receives the detection signal from the pressure sensor 12 indicating the human 300 sitting on the seat, even when the control unit 20 receives the detection signal from the door sensor 13 indicating the door 203 closed, the control unit 20 does not cause the timer 0 to start counting, unless the control unit 20 receives the detection signal from the pressure sensor 12 indicating the exiting of the human 300.

Therefore, the absence of the human in the restroom stall 200 is appropriately determined by checking both of the detection signal from the door sensor 13 and the detection signal from the pressure sensor 12.

Similar to the modification 1 to the second embodiment, if no human is present in the restroom stall 200, ultraviolet light may be radiated only during a predetermined time T2 in the restroom stall 200, i.e., the irradiation with ultraviolet light in the restroom stall 200 may be stopped when the predetermined time T2 has elapsed since the time R3 in FIG. 10.

(Other Modifications)

In each of the above-described embodiments, the case in which the inactivation device 100 is disposed in the restroom stall is described; however, the present invention is not limited to the above-described embodiments. The inactivation device 100 can be particularly disposed in confined spaces in facilities where people frequently gather, such as hospital rooms, elevators, and conference rooms.

The timing at which the inactivation device 100 irradiates a human with ultraviolet light can be any timing during a period when the human is present in the enclosed space. When a timing at which harmful microorganisms or viruses is likely to spread is detectable during the period when the human is present in the enclosed space, it is preferable that ultraviolet light be radiated at that timing.

In each of the above-described embodiments, the case in which the inactivation device 100 is disposed in the enclosed space where a human can enter or leave is described; however, the enclosed space can be a space where an animal other than a human can enter or leave.

In each of the above-described embodiments, ultraviolet light is radiated to the human or the space including the human just for the predetermined time T1; however, in the case of a light source repeatedly operating the light emission and the non-light emission, the sum of the light emission operation time may be the predetermined time T1.

For example, in the case of controlling the power to the excimer lamp to repeat the light emission operation and the pause, with the light emission operation time of the excimer lamp being between 10 milliseconds and 1000 milliseconds, and the subsequent pause time being between 10 milliseconds and 10 seconds, the predetermined time T1 is a sum of the light emission operation time.

Specifically, in the case that the light emission operation time of the KrCl excimer lamp is 100 milliseconds, the pause time is 100 milliseconds and the predetermined time T1 is 30 seconds, for example, the light emission operation count of the KrCl reaches 300 and the operation time of the Kr excimer lamp including the pause time reaches 60 seconds.

In other word, in the case that a light source continuously operates, the irradiation period of ultraviolet light to the human or the space including the human is the predetermined time T1; however, in the case that the light source intermittently operates including a pause time, the irradiation period of ultraviolet light becomes longer than the predetermined time T1.

In the case that the space including the human is, for example, a restroom stall, the light source can be controlled to intermittently operate so that the irradiation period of ultraviolet light is set to be longer, thereby increasing the chance of radiating ultraviolet light when, for example, the dispersion of bacteria and/or viruses takes place upon defecation or splash.

In the above-described case in which the light emission operation time is set to between 10 milliseconds and 1000 milliseconds and the pause time is set to between 10 milliseconds and 10 seconds, a shutter for shielding light is controlled to open and close in the case of low-pressure mercury lamp as described above; however, in some cases, the opening and closing of the shutter needs to be carried out with high speed, which is difficult to achieve.

Hence, a suitable ultraviolet light source is capable of repeating the ultraviolet light emitting operation and the pause time using the power control.

Examples of such light sources are excimer lamps (KeCl excimer lamps) and solid state light sources (light-emitting diodes (LED), laser diodes (LD)) as described above.

The inactivation device and inactivation method of the present invention are capable of providing sterilization intrinsic to ultraviolet light and inactivation of viruses while preventing the radiation of ultraviolet light from adversely affecting humans. In particular, unlike the conventional ultraviolet light sources, the inactivation device and inactivation method of the present invention have the feature of being able to be used in manned environments and are capable of utilizing this feature to reduce and sterilize viruses in the air and the surface of components placed in an enclosed space when the device is installed in the enclosed space such as a facility or a vehicle where people or animal are present and irradiates the entire enclosed space with its ultraviolet light.

This feature corresponds to the Goal 3 of the United Nations-led Sustainable Development Goals (SDGs) "Ensure healthy lives and promote well-being for all at all ages", and also significantly contributes to achieving the Target 3.3 "By 2030, end the epidemics of AIDS, tuberculosis, malaria and neglected tropical diseases and combat hepatitis, water-borne diseases and other communicable diseases".

The certain embodiments have been described in the foregoing; however, they are merely illustrative and are not intended to limit the scope of the present invention. The devices and methods described herein may be embodied in forms other than those described above. In addition, omissions, substitutions, and modifications may be made to the above-described embodiments as appropriate without departing from the scope of the present invention. Such omissions, substitutions and modifications are encompassed in the scope of the claims and their equivalents, and belong to the technical scope of the present invention.

Reference Signs List

| | |
|---|---|
| 10A, 10B | UV irradiation unit |
| 11 | Motion sensor |
| 12 | Pressure sensor |
| 13 | Door sensor |
| 20 | Control unit |
| 100 | Inactivation device |
| 200 | Enclosed space (restroom stall) |
| 201 | Ceiling |
| 202 | Wall |
| 203 | Door |
| 300 | Human |

**Claims**

1. An inactivation device for inactivating microorganisms and/or viruses that are harmful to a human body, the inactivation device comprising:

   an ultraviolet light irradiation unit that irradiates an enclosed space where a human can enter and leave with light containing ultraviolet light having a wavelength of inactivating the microorganisms and/or viruses that are harmful to the human body;
   a sensor that detects presence of the human in the enclosed space; and
   a control unit that controls the irradiation and non-irradiation with the light from the ultraviolet light irradiation unit based on a detection signal from the sensor;
   wherein the control unit controls the ultraviolet light irradiation unit to irradiate a space including the human with the light for a predetermined time in accordance with the wavelength of the ultraviolet light contained in the light radiated from the ultraviolet light irradiation unit, during a period in which the human is determined to be present in the enclosed space based on the detection signal from the sensor.

2. The inactivation device according to claim 1, wherein if it is determined that no human is present in the enclosed space based on the detection signal from the sensor, the control unit controls the ultraviolet irradiation unit to irradiate the enclosed space where no human is present with the light.

3. The inactivation device according to claim 2, wherein the control unit controls the ultraviolet irradiation unit to irradiate the enclosed space where no human is present with the light for a predetermined time, and subsequently controls the ultraviolet irradiation unit to stop radiating the light.

4. The inactivation device according to any one of claims 1 to 3, wherein the sensor includes a first sensor that detects the human entering or leaving the enclosed space, and the control unit controls the ultraviolet light irradiation unit to irradiate the space including the human with the light for the predetermined time after the first sensor detects that the human has entered the enclosed space.

5. The inactivation device according to claim 4, wherein the first sensor is at least one of a motion sensor that detects the presence or absence of the human in the enclosed space and a door sensor that detects opening or closing of a door of the enclosed space.

6. The inactivation device according to any one of claims 1 to 3, wherein the sensor includes a second sensor that detects the presence of the human at a predetermined position in the enclosed space, and the control unit controls the ultraviolet light irradiation unit to irradiate the space including the human with the light for the predetermined time after the second sensor detects the presence of the human at the predetermined position in the enclosed space.

7. The inactivation device according to any one of claims 1 to 3, wherein the sensor includes a first sensor that detects the human entering or leaving the enclosed space and a second sensor that detects the presence of the human at a predetermined position in the enclosed space, the control unit controls the ultraviolet light irradiation unit to stop radiation of the light when the first sensor detects that the human has entered the enclosed space and start irradiating the space including the human with the light for the predetermined time after the second sensor detects the presence of the human at the predetermined position in the enclosed space.

8. The inactivation device according to claim 7, wherein the first sensor is a door sensor that detects opening or closing of a door of the enclosed space, and
   if the second sensor does not detect the presence of the human at the predetermined position in the enclosed space, the control unit controls the ultraviolet light irradiation unit to irradiate the space where no human is present with the light after a predetermined time has elapsed since the first sensor detects the opening or closing of the door.

9. The inactivation device according to claims 6, wherein the enclosed space is a restroom stall and the second sensor is a pressure sensor provided in a toilet seat.

10. The inactivation device according to claims 7, wherein the enclosed space is a restroom stall and the second sensor is a pressure sensor provided in a toilet seat.

11. The inactivation device according to claims 6, wherein the ultraviolet irradiation unit is mounted at a position such that the light is radiated to the human present at the predetermined position from a back of a head of the human.

12. The inactivation device according to claims 7, wherein the ultraviolet irradiation unit is mounted at a position such that the light is radiated to the human present at the predetermined position from a back of a head of the human.

13. The inactivation device according to any one of claims 1 to 3, wherein the ultraviolet irradiation unit is mounted at a position at which the light is radiated downward from an upper area of the enclosed space.

14. The inactivation device according to any one of claims 1 to 3, wherein the predetermined time T1 (seconds) is set to satisfy a formula: $T1 \leq D_{max} / (W \times N)$
where $D_{max}$ (mJ/cm$^2$) is an amount of maximum allowable ultraviolet light exposure to the human body in one day, W (mW/cm$^2$) is irradiance of the ultraviolet light radiated on a surface of the human body, and N is the number of times the same person enters the enclosed space per day.

15. The inactivation device according to claim 14, wherein an ultraviolet light emission operation of the ultraviolet light irradiation unit includes repetition of ultraviolet light emission and subsequent pause; and
a total time of the ultraviolet light emission in the repeated ultraviolet light emission operations of twice or more is set to be the time T1.

16. The inactivation device according to claim 15, wherein time of the ultraviolet light emission is between 10 milliseconds and 1000 milliseconds, and time of the pause is between 10 milliseconds and 10 seconds.

17. The inactivation device according to any one of claims 1 to 3, wherein the ultraviolet light irradiation unit includes a KrCl excimer lamp that radiates the ultraviolet light having a center wavelength of 222 nm.

18. The inactivation device according to claim 16, wherein the ultraviolet light irradiation unit includes a light-emitting diode (LED) that radiates the ultraviolet light or a laser diode (LD) that radiates the ultraviolet light.

19. The inactivation device according to any one of claims 1 to 3, wherein ultraviolet light contained in the light radiated from the ultraviolet light irradiation unit only has a wavelength band ranging from 190 nm to 235 nm.

20. An inactivation method of inactivating microorganisms and/or viruses harmful to a human body, the inactivation method comprising:

detecting presence of a human in an enclosed space that the human can enter and leave, with a sensor; and controlling an ultraviolet light irradiation unit that irradiates an enclosed space with light containing ultraviolet light having a wavelength suitable for inactivating microorganisms and/or viruses harmful to the human body to perform irradiation and non-irradiation with the light, and irradiating a space including the human with the light for a predetermined time in accordance with the wavelength of the ultraviolet light contained in the light during a period in which the human is determined to be present in the enclosed space.

FIG. 1

FIG. 2

```
                    ┌─────────────┐
                    │    START    │
                    └──────┬──────┘
                           │
        ┌──────────────────┼──────────────────────────────┐
        │         ╱────────┼────────╲              S1      │
   No   │◄───────< Presence of human  >                    │
        │         ╲  detected?       ╱                     │
        │          ╲───────┬────────╱                      │
        │                  │ Yes                           │
        │          ┌───────┴────────────┐       S2         │
        │          │ Start counting on  │                  │
        │          │      timer 1       │                  │
        │          └───────┬────────────┘                  │
        │                  │                               │
        │         ╱────────┼────────╲              S3      │
   No   │◄───────< Predetermined time >                    │
        │         ╲  T1 elapsed?      ╱                     │
        │          ╲───────┬────────╱                      │
        │                  │ Yes                           │
        │          ┌───────┴────────────┐       S4         │
        │          │ Terminate counting │                  │
        │          │    on timer 1      │                  │
        │          └───────┬────────────┘                  │
        │                  │                               │
        │          ┌───────┴────────────┐       S5         │
        │          │  Stop UV radiation │                  │
        │          └───────┬────────────┘                  │
        │                  │                               │
        │         ╱────────┼────────╲              S6      │
   No   │◄───────<  Exiting of human  >                    │
        │         ╲  detected?       ╱                     │
        │          ╲───────┬────────╱                      │
        │                  │ Yes                           │
        │          ┌───────┴────────────┐       S7         │
        │          │Starting UV radiation│                 │
        │          └───────┬────────────┘                  │
        │                  │                               │
        └──────────────────┴───────────────────────────────┘
```

FIG. 3

## FIG. 4

```
                    ┌─────────────┐
                    │    START    │
                    └──────┬──────┘
                           │
   ┌──────────────────────────────────────────┐
No │         Presence of human                 │  S1
◄──┤         detected?                          │
   └──────────────────┬───────────────────────┘
                      │ Yes
          ┌───────────────────────────┐
          │    Start UV radiation      │  S11
          └───────────┬───────────────┘
                      │
          ┌───────────────────────────┐
          │  Start counting on timer 1 │  S2
          └───────────┬───────────────┘
                      │
   ┌──────────────────────────────────────────┐
No │      Predetermined time T1                 │  S3
◄──┤      elapsed?                              │
   └──────────────────┬───────────────────────┘
                      │ Yes
          ┌───────────────────────────┐
          │ Terminate counting on timer 1 │  S4
          └───────────┬───────────────┘
                      │
          ┌───────────────────────────┐
          │    Stop UV radiation       │  S5
          └───────────┬───────────────┘
                      │
   ┌──────────────────────────────────────────┐
No │         Exiting of human                   │  S6
◄──┤         detected?                          │
   └──────────────────┬───────────────────────┘
                      │ Yes
          ┌───────────────────────────┐
          │    Start UV radiation      │  S7
          └───────────┬───────────────┘
                      │
          ┌───────────────────────────┐
          │  Start counting on timer 2 │  S12
          └───────────┬───────────────┘
                      │
   ┌──────────────────────────────────────────┐
No │      Predetermined time T2                 │  S13
◄──┤      elapsed?                              │
   └──────────────────┬───────────────────────┘
                      │ Yes
          ┌───────────────────────────┐
          │    Stop UV radiation       │  S14
          └───────────────────────────┘
```

FIG. 5

FIG. 6

```
                    ┌──────────────┐
                    │    START     │
                    └──────┬───────┘
                           │
    ┌──────────────────────┴──────────────────────┐
    │                      ↓                        │
    │  ┌─────────────────────────────────┐         │
 No─┤  │  Presence of human detected?    │ S21     │
    │  └─────────────────────────────────┘         │
    │                  │ Yes                         │
    │  ┌─────────────────────────────────┐         │
    │  │       Stop UV radiation         │ S22     │
    │  └─────────────────────────────────┘         │
    │                  │                            │
    │  ┌─────────────────────────────────┐         │
 No─┤  │  Human sitting on the           │ S23     │
    │  │  seat detected?                 │         │
    │  └─────────────────────────────────┘         │
    │                  │ Yes                         │
    │  ┌─────────────────────────────────┐         │
    │  │       Start UV radiation        │ S24     │
    │  └─────────────────────────────────┘         │
    │                  │                            │
    │  ┌─────────────────────────────────┐         │
    │  │    Start counting on timer 1    │ S25     │
    │  └─────────────────────────────────┘         │
    │                  │                            │
    │  ┌─────────────────────────────────┐         │
 No─┤  │  Predetermined time T1          │ S26     │
    │  │  elapsed?                       │         │
    │  └─────────────────────────────────┘         │
    │                  │ Yes                         │
    │  ┌─────────────────────────────────┐         │
    │  │  Terminate counting on timer 1  │ S27     │
    │  └─────────────────────────────────┘         │
    │                  │                            │
    │  ┌─────────────────────────────────┐         │
    │  │       Stop UV radiation         │ S28     │
    │  └─────────────────────────────────┘         │
    │                  │                            │
    │  ┌─────────────────────────────────┐         │
 No─┤  │  Exiting of human detected?     │ S29     │
    │  └─────────────────────────────────┘         │
    │                  │ Yes                         │
    │  ┌─────────────────────────────────┐         │
    │  │       Start UV radiation        │ S30     │
    │  └─────────────────────────────────┘         │
    └──────────────────────────────────────────────┘
```

FIG. 7

Motion sensor

Pressure sensor

Timer 1

Ultraviolet
light

T1

P    Q                          R

FIG. 8

```
                    ┌─────────────────┐
                    │      START      │
                    └─────────────────┘
                             │
    ┌────────────────────────┼───────────────────────────┐
    │         ┌──────────────▼──────────────┐            │
  No◄─────────┤   Presence of human          │       S21  │
    │         │   detected?                  │            │
    │         └──────────────┬──────────────┘            │
    │                        │ Yes                        │
    │         ┌──────────────▼──────────────┐            │
  No◄─────────┤  Human sitting on the        │       S23  │
    │         │  seat detected?              │            │
    │         └──────────────┬──────────────┘            │
    │                        │ Yes                        │
    │         ┌──────────────▼──────────────┐            │
    │         │      Start UV radiation      │       S24  │
    │         └──────────────┬──────────────┘            │
    │         ┌──────────────▼──────────────┐            │
    │         │   Start counting on timer 1  │       S25  │
    │         └──────────────┬──────────────┘            │
    │         ┌──────────────▼──────────────┐            │
  No◄─────────┤   Predetermined time T1      │       S26  │
    │         │   elapsed?                   │            │
    │         └──────────────┬──────────────┘            │
    │                        │ Yes                        │
    │         ┌──────────────▼──────────────┐            │
    │         │  Terminate counting on timer 1│      S27  │
    │         └──────────────┬──────────────┘            │
    │         ┌──────────────▼──────────────┐            │
    │         │      Stop UV radiation       │       S28  │
    │         └──────────────┬──────────────┘            │
    │         ┌──────────────▼──────────────┐            │
  No◄─────────┤    Exiting of human          │       S29  │
    │         │    detected?                 │            │
    │         └──────────────┬──────────────┘            │
    │                        │ Yes                        │
    │         ┌──────────────▼──────────────┐            │
    │         │      Start UV radiation      │       S30  │
    │         └──────────────┬──────────────┘            │
    │         ┌──────────────▼──────────────┐            │
    │         │   Start counting on timer 2  │       S31  │
    │         └──────────────┬──────────────┘            │
    │         ┌──────────────▼──────────────┐            │
  No◄─────────┤   Predetermined time T2      │       S32  │
    │         │   elapsed?                   │            │
    │         └──────────────┬──────────────┘            │
    │                        │ Yes                        │
    │         ┌──────────────▼──────────────┐            │
    │         │      Stop UV radiation       │       S33  │
    │         └──────────────┬──────────────┘            │
    └────────────────────────┘                           │
                                                          │
```

25

FIG. 9

FIG. 10

## INTERNATIONAL SEARCH REPORT

| | International application No. |
|---|---|
| | PCT/JP2021/008462 |

### A. CLASSIFICATION OF SUBJECT MATTER
Int.Cl. A47K17/00(2006.01)i, E03D9/00(2006.01)i, A61L2/10(2006.01)i
FI: A61L2/10, A47K17/00, E03D9/00

According to International Patent Classification (IPC) or to both national classification and IPC

### B. FIELDS SEARCHED
Minimum documentation searched (classification system followed by classification symbols)
Int.Cl. A47K17/00, E03D9/00, A61L2/10

Documentation searched other than minimum documentation to the extent that such documents are included in the fields searched
Published examined utility model applications of Japan    1922–1996
Published unexamined utility model applications of Japan  1971–2021
Registered utility model specifications of Japan         1996–2021
Published registered utility model applications of Japan 1994–2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y<br>A | JP 2011-98156 A (MIURA KK) 19 May 2011 (2011-05-19), claims 1, 5, paragraph [0057], fig. 4 | 1-6, 11-20<br>7-10 |
| Y | JP 2018-114209 A (NIKKISO CO., LTD.) 26 July 2018 (2018-07-26), paragraph [0027] | 1-6, 11-20 |
| Y | WO 2017/183538 A1 (MITSUBISHI ELECTRIC CORPORATION) 26 October 2017 (2017-10-26), paragraph [0047] | 6, 11 |
| Y | WO 2019/186880 A1 (SUN ENERGY CORP.) 03 October 2019 (2019-10-03), paragraphs [0085], [0136], [0137], [0165] | 14-16 |
| Y | JP 2014-508612 A (THE TRUSTEES OF COLUMBIA UNIVERSITY IN THE CITY OF NEW YORK) 10 April 2014 (2014-04-10), paragraphs [0009], [0024] | 17-19 |

☐ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 April 2021 | 27 April 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

| INTERNATIONAL SEARCH REPORT | International application No. |
|---|---|
| Information on patent family members | PCT/JP2021/008462 |

| | | |
|---|---|---|
| JP 2011-98156 A | 19 May 2011 | (Family: none) |
| JP 2018-114209 A | 26 July 2018 | US 2018/0207304 A1<br>paragraph [0037] |
| WO 2017/183538 A1 | 26 October 2017 | CN 109073251 A |
| WO 2019/186880 A1 | 03 October 2019 | JP 6490318 B1 |
| JP 2014-508612 A | 10 April 2014 | US 2015/0073396 A1<br>paragraphs [0009], [0033]<br>US 2016/0107000 A1<br>US 2018/0169279 A1<br>US 2019/0160305 A1<br>US 2019/0381336 A1<br>US 2019/0388706 A1<br>US 2020/0085984 A1<br>US 2020/0215215 A1<br>WO 2012/122210 A1<br>EP 2683442 A1 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017528258 A
- US 20100032859 A